# EUROPÄISCHE PATENTSCHRIFT

(11) **EP 1 997 422 B1**
(45) Veröffentlichungstag und Bekanntmachung des Hinweises auf die Patenterteilung: **13.11.2013**
(21) Anmeldenummer: 08013109.7
(22) Anmeldetag: 01.09.2004
(51) Int. Cl.: A61B 3/10, A61B 3/103

(54) **Schnelle Wellenfrontmessung**
Quick wave front measurement
Rapide mesure de fronts d'ondes

(43) Veröffentlichungstag der Anmeldung: 03.12.2008
(62) Teilanmeldung aus: 04020783.9
(73) Patentinhaber: WaveLight GmbH, 91058 Erlangen (DE)
(72) Erfinder: Gorschboth, Claudia, 90411 Nürnberg (DE); Donitzky, Christof, 90542 Eckental (DE)
(74) Vertreter: von Hellfeld, Axel

(56) Entgegenhaltungen:
- DE-A- 10 154 194
- US-A1- 2002 008 848
- US-A1- 2003 071 969
- US-B1- 6 199 986
- US-B1- 6 338 559
- HOFER H ET AL: "DYNAMICS OF THE EYE'S WAVE ABERRATION" JOURNAL OF THE OPTICAL SOCIETY OF AMERICA - A, OPTICAL SOCIETY OF AMERICA, WASHINGTON, US, Bd. 18, Nr. 3, März 2001 (2001-03), Seiten 497-506, XP001041247 ISSN: 1084-7529

## Beschreibung

### Gebiet der Erfindung

Die vorliegende Erfindung betrifft im Allgemeinen die Erfassung von Wellenfronten zur Bestimmung der Aberration eines Auges und insbesondere die schnelle, dynamische Erfassung von Wellenfronten, um nichtlineare Abbildungseigenschaften einer Linse für ein Auge zu bestimmen.

### Hintergrund der Erfindung

In der Ophthalmologie sind Systeme zur Messung der Wellenfrontaberration eines Auges bekannt. In der Praxis haben sich zur Wellenfrontmessung sogenannte Hartmann-Shack-Sensoren etabliert. Bei diesen Sensoren wird die vermessende Wellenfront von einem Mikrolinsenarray auf einen lichtempfindlichen Detektor als Punktmuster abgebildet. Bei Abweichungen der detektierten Wellenfront von einer idealen Wellenfront aufgrund der Aberration des Auges kann diese aus dem detektierten Punktmuster berechnet werden. Hierfür wird ein schmales Lichtbündel auf das zu überprüfende Auge gerichtet und Licht nach Wechselwirkung mit dem Auge auf den Hartmann-Shack-Sensor abgebildet.

Insbesondere im Bereich der refraktiven Chirurgie kommen Systeme zur Messung von Wellenfrontaberrationen zum Einsatz. Dabei ist es bekannt, Systeme zu verwenden, die die Aberration eines Auges messen, wenn das Auge einen in einer festgelegten Entfernung erscheinenden Stimulus fixiert. Neuere Systeme erlauben die Berechung der Aberration eines Auges durch Erfassung von Aberrationen bei Fixierung eines Stimulus, der in unterschiedlichen Entfernungen wahrgenommen wird. Die Berechnung der Aberration erfolgt auf der Grundlage der für unterschiedliche Stimuli erfassten Aberrationen. Die DE 101 54 194 beschreibt die Messung von Wellenfronten, die von einer Augenlinse erzeugt werden mit einer Strahlungsquelle zur Abgabe von auf die Linse zu richtender Messstrahlung und einer Sensoreinrichtung zur Erfassung von Wellenfronten von einfallender Messstrahlung nach Wechselwirkung mit der Linse, und mit einer Sensoreinrichtung zur Erfassung der einfallenden Messstrahlung mit einer Abtastfrequenz, die wenigstens so groß ist wie die Frequenz, mit der Wellenfrontänderungen in der einfallenden Messstrahlung auftreten.

Die US 2002/0008848 A1 betrifft eine Einrichtung zum Untersuchen von Blutgefäßen im Auge. Mittels des Dopplereffekts wird die Geschwindigkeit des in einem Blutgefäß strömenden Bluts bestimmt. Das Blutgefäß wird mittels eines Zeilensensors ausgewertet und das Dopplersignal wird mittels Photomultiplizierer erfasst. Ferner wird eine Filtereinheit verwendet, die die Intensität des dem Auge des Patienten zugeführten Lichtstrahls verändert. Ferner ist vor dem Zeilensensor ein Bildverstärker angeordnet, der ein Bildsignal verstärkt, wenn ein Filter mit niedriger Durchlässigkeit in den Strahlengang des Beobachtungsstrahls geschaltet wird. Jedoch verschlechtert der Bildverstärker den Signal-Rausch-Abstand.

### Aufgabe der Erfindung

Aufgabe der vorliegenden Erfindung ist es, Lösungen bereitzustellen, die eine verbesserte und umfassenderere Messung von Wellenfronten im Allgemeinen zur Bestimmung der Aberration eines Auges und insbesondere zur Bestimmung optischer Eigenschaften von Linsen für ein Auge ermöglichen.

### Zusammenfassung der Erfindung

Zur Lösung der Aufgabe stellt die vorliegende Erfindung ein Verfahren, eine Vorrichtung sowie Verwendungen gemäss den unabhängigen Ansprüchen bereit. Vorteilhafte Weiterbildungen erfindungsgemässer Lösungen sind in den abhängigen Ansprüchen definiert.

Gemäss Anspruch 1 stellt die vorliegende Erfindung eine Vorrichtung zur Messung von Wellenfronten einer Linse für ein Auge bereit. Dabei kann unter einer Linse für ein Auge insbesondere die Linse eines Auges, eine Kontaktlinse oder eine intraokulare Linse verstanden werden.

Die Vorrichtung umfasst eine Strahlungsquelle zur Abgabe von Messstrahlung, die auf die Linse zu richten ist. Ferner ist eine Sensoreinrichtung vorhanden, um Wellenfronten in zu der Sensoreinrichtung gelangender Messstrahlung zu erfassen, die sich durch die Messstrahlung der Strahlungsquelle nach Wechselwirkung mit der Linse ergibt.

Insbesondere ist die Sensoreinrichtung so ausgeführt, dass sie die einfallende Messstrahlung mit einer Abtastfrequenz nach Wellenfronten abtastet, die wenigstens so gross wie die Frequenz ist, mit der Wellenfrontänderungen in der einfallenden Messstrahlung auftreten.

Dadurch wird beispielsweise erreicht, dass auch in dynamischen Sehsituationen die Aberration eines Auges korrekt bestimmt werden kann. Des Weiteren können auf diese Weise Akkommodationsvorgänge eines Auges in bisher nicht bekanntem Umfang analysiert werden. Des Weiteren wird dadurch ermöglicht, wie im Folgenden detaillierter ausgeführt, die chromatische Aberration einer Linse für ein Auge zu bestimmen.

Die Strahlungsquelle kann so ausgeführt sein, dass sie eine Messstrahlung abgibt, deren Wellenlänge sich mit einer Strahlungsabgabefrequenz ändert. Darunter ist insbesondere zu verstehen, dass sich die Wellenlänge der von der Strahlungsquelle abgegebenen Messstrahlung nach einer vorgegebenen Zeitdauer ändert. Vorzugsweise ist dabei die Abtastfrequenz der Sensoreinrichtung wenigstens so gross wie die Strahlungsabgabefrequenz.

Die Vorrichtung kann ferner eine Stimuluserzeugungseinrichtung umfassen, um einen Stimulus zu erzeugen, der dynamische Änderungen der Linse bewirken soll. Beispiele für dynamische Änderungen umfassen Änderungen der Linse aufgrund von Akkommodationen. Vorzugsweise ist hierbei die Abtastfrequenz wenigstens so gross wie die Frequenz der zu bewirkenden Änderungen. Z. B. kann hierbei eine Abtastfrequenz gewählt werden, die wenigstens so gross wie die Frequenz ist, mit der der Stimulus variiert wird.

Vorzugsweise umfasst die Sensoreinrichtung einen optischen Sensor, der beispielsweise ein CMOS-Sensor sein kann.

Die Sensoreinrichtung kann eine Abtastfrequenz von wenigstens 70 Hertz, 100 Hertz oder grösser aufweisen.

Die Sensoreinrichtung kann eine Verstärkungseinrichtung aufweisen, um auf die Sensoreinrichtung einfallende Messstrahlung, d.h. Messstrahlung der Strahlungsquelle nach Wechselwirkung mit der Linse, zu verstärken. Beispielsweise kann die Verstärkungseinrichtung einen Bildverstärker umfassen.

Vorzugsweise ist die Verstärkungseinrichtung so angeordnet, dass die Verstärkung der einfallenden Messstrahlung vor deren Erfassung mit der Abtastfrequenz erfolgt.

Die Sensoreinrichtung kann eine Linsenanordnung umfassen, die beispielsweise in Abhängigkeit einer gewünschten Auflösung und/oder einer gewünschten Dynamik der Sensoreinrichtung insgesamt ausgelegt ist.

Vorzugsweise ist die Strahlungsquelle so ausgeführt, dass deren Messstrahlung eine vorgegebene maximale Strahlungsleistung aufweist, die für die Linse vorgegeben ist. Insbesondere wenn es sich bei der Linse um die Linse eines Auges handelt, werden durch diese Ausführungsform unerwünschte Einflüsse aufgrund der Messstrahlung vermieden.

Die Strahlungsquelle kann wenigstens eine Quelle für Laserstrahlung umfassen, die vorzugsweise eine fest vorgegebene Wellenlänge abgibt. Beispielsweise kann die wenigstens eine Laserstrahlenquelle ein Laser, eine Laserdiode oder eine Superluminiszenzdiode (SLD) sein.

Die Strahlungsquelle kann ausgangsseitig mit einer Schalteinrichtung verbunden sein, die mit einer Schaltfrequenz betrieben werden kann. Diese Ausführungsform ist insbesondere dann bevorzugt, wenn mehr als eine Laserstrahlenquelle verwendet wird, um Laserstrahlung der unterschiedlichen Laserstrahlenquellen gemäss der Schaltfrequenz der Schalteinrichtung auf die Linse zu richten. Bei Verwendung von nur einer Laserstrahlenquelle kann deren Laserstrahlung gemäss der Schaltfrequenz, beispielsweise zu vorbestimmten Zeitpunkten, in vorbestimmten, regelmässigen oder unregelmässigen Zeitabständen, auf die Linse gerichtet werden.

Vorzugsweise umfasst die Schalteinrichtung einen Faserschalter oder Faserkoppler.
Gemäss einer Ausführungsform ist die Strahlungsquelle eingerichtet, Messstrahlung mit einer, zwei oder mehreren Wellenlängen im Bereich zwischen 400 nm und 1000 nm zu erzeugen. Dadurch ist es beispielsweise möglich, schnelle Wellenfrontmessungen bei verschiedenen, diskreten Wellenlängen durchzuführen, die über den gesamten sichtbaren Bereich bis in den Infrarotbereich hinein reichen.

Als Stimuluserzeugungseinrichtung ist vorzugsweise ein Badaloptometer vorgesehen.

Des Weiteren stellt die vorliegende Erfindung ein Verfahren zur Messung von Wellenfronten einer Linse für ein Auge bereit, das die Schritte umfasst, einen Messstrahl auf die Linse zu richten und Wellenfronten von Messstrahlung nach Wechselwirkung mit der Linse zu erfassen, wobei die Erfassung der Messstrahlung nach Wechselwirkung mit der Linse mit einer Abtastfrequenz erfolgt, die wenigstens so gross wie die Frequenz ist, mit der Wellenfrontänderungen in der erfassten Messstrahlung auftreten.

Des Weiteren stellt die vorliegende Erfindung Verwendungen der oben beschriebenen Vorrichtung in einer ihrer Ausführungsformen bereit, um dynamische Akkommodationsänderungen der Linse eines Auges, chromatischen Aberrationen eines Auges oder die Dispersion einer Kontaktlinse oder einer intraokularen Linse für ein Auge oder optische Variationen des Tränenfilms zu messen.

### Kurzbeschreibung der Zeichnungen

Bei der folgenden Beschreibung wird auf die beigefügten Zeichnungen Bezug genommen, die zeigen:
Fig. 1 eine schematische Darstellung einer bevorzugten Ausführungsform der erfindungsgemässen Vorrichtung zur Messung von Wellenfronten unter dynamischen Sehbedingungen,
Fig. 2 eine schematische Darstellung einer bevorzugten Ausführungsform der erfindungsgemässen Vorrichtung zur Erfassung von Wellenfronten für verschiedene Wellenlängen unter einer statischen Sehbedingung, und
Fig. 3 eine schematische Darstellung einer bevorzugten Ausführungsform zur Erfassung von Wellenfronten einer Kontaktlinse oder einer intraokularen Linse für ein Auge.

### Beschreibung bevorzugter Ausführungsformen

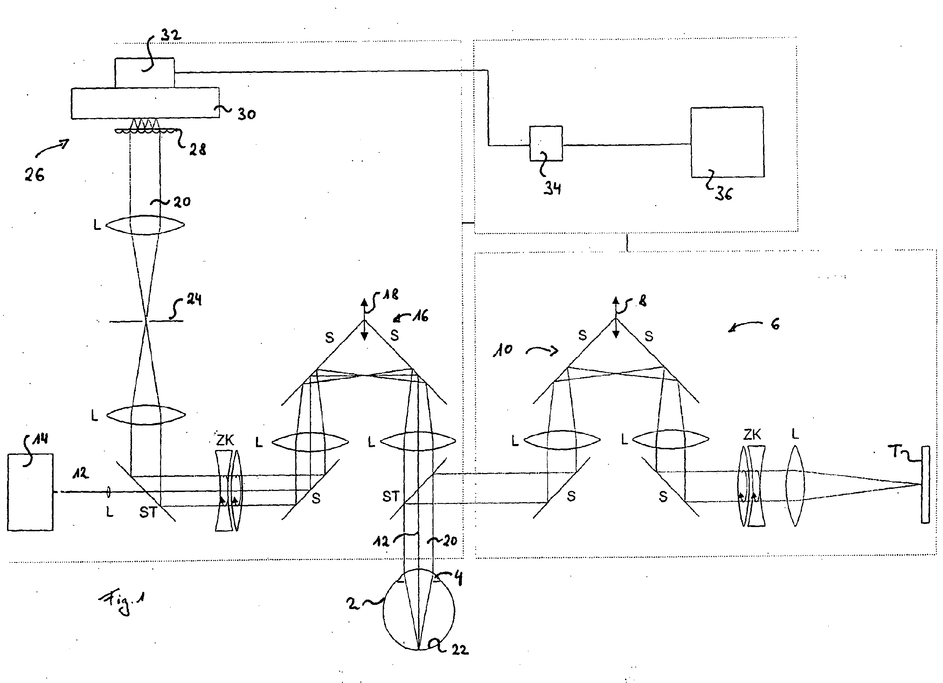

In Fig. 1 bis 3 sind Linsen L, Spiegel S, Strahlteiler ST und Zylinderkompensatoren ZK mit den genannten Bezugszeichen ohne weitere Differenzierungen angegeben.

Die in Fig. 1 schematisch dargestellte Ausführungsform dient zur Erfassung von Wellenfronten eines Auges 2 und insbesondere einer Linse 4 des Auges 2. Insbesondere dient diese Ausführungsform zur Messung von Wellenfronten unter dynamischen Sehbedingungen für das Auge 2 in Form von dynamischen Akkommodationsvorgängen.

Um dynamische Akkommodationen des Auges 2 bzw. der Linse 4 zu bewirken, wird ein in unterschiedlichen Entfernungen erscheinender Stimulus bereitgestellt. Hierfür wird über eine im ganzen mit 6 bezeichnete Stimuluserzeugungseinrichtung in Form eines Badaloptometers ein als Stimulus wahrnehmbares Bild eines Ziels oder Targets T bereitgestellt. Das Bild des Targets T wird dem Auge über Linsen L, einen Zylinderkompensator ZK, Spiegel S und einen Strahlteiler ST bereitgestellt. Um den Stimulus bzw. das Bild des Targets T in für das Auge unterschiedlichen Entfernungen erscheinen zu lassen, umfasst die Stimuluserzeugungseinrichtung 6 eine in Richtung des Pfeils 8 bewegbare Spiegel- oder Prismenanordnung 10.

Zur Messung wird Messstrahlung 12, hier in Form von Laserstrahlung eines Lasers 14 verwendet. Der Laser 14 kann beispielsweise ein Laser, eine Laserdiode oder eine Superluminiszenzdiode (SLD) sein und Messstrahlung 12 abgeben, die eine Wellenlänge im Bereich sichtbaren Lichts bis in den Infrarotbereich hinein aufweist.

Die Messstrahlung wird über Linsen L, eine Zylinderkompensator ZK und Spiegel S dem Auge zugeführt. Diese Anordnung optischer Komponenten umfasst eine Spiegel- oder Prismenanordnung 16, die in Richtung des Pfeils 18 bewegbar ist. Bewegungen der Anordnungen 10 und 16 erfolgen im Allgemeinen in Abhängigkeit von einander, um einerseits das Bild des Targets T in unterschiedlichen Entfernungen erscheinen zu lassen und andererseits die sich daraus ergebenen Auswirkungen für die Messstrahlung und/oder das Auge hinsichtlich der Messstrahlung zu berücksichtigen.

Messstrahlung 20, die sich nach Wechselwirkung der Messstrahlung 12 mit dem Auge 2 (insbesondere Wechselwirkungen aufgrund von Durchtritten durch die Linse 4 und Reflexion an der Retina 22 des Auges 2) ergibt, wird über Linsen L, Spiegel S, einen Zylinderkompensator ZK und eine Blende 24 zu einer im ganzen mit 26 bezeichneten Sensoreinrichtung geführt. Die Sensoreinrichtung 26 dient zur Erfassung von Wellenfronten der Messstrahlung 20.

Die Sensoreinrichtung 26 umfasst eine Linsenanordnung 28, beispielsweise in Form eines Mikrolinsenarrays. Die Linsenanordnung 28 kann zum Beispiel Linsen mit einem Durchmesser von 650 µm und einer Brennweite von 30 mm aufweisen.

Der Linsenanordnung 28 nachgeordnet ist eine Verstärkungseinrichtung 30. Die Verstärkungseinrichtung 30, beispielsweise in Form eines Bildverstärkers, verstärkt die Messstrahlung 20 nach Abbildung durch die Linsenanordnung 28 auf entsprechende Bereiche der Verstärkungseinrichtung 30.

Die Verwendung der Verstärkungseinrichtung 30 ermöglicht es, als Messstrahlung 12 eine Strahlung zu verwenden, deren Strahlungsleistung für das Auge 2 maximale Grenzwerte nicht überschreitet. Dies führt im Allgemeinen zu Messstrahlung 12 relativ geringer Strahlungsleistung. Nach Wechselwirkung der Messstrahlung 12 mit dem Auge 2 ergibt sich eine Messstrahlung 20 noch geringerer Strahlungsleistung. Üblicherweise wird dieses Problem dadurch gelöst, dass einerseits möglichst lange Belichtungszeiten und andererseits möglichst lichtempfindliche Sensoren verwendet werden. Lange Belichtungszeiten verbieten es, dynamische Sehvorgänge zu analysieren. Lichtempfindliche Sensoren stellen nur geringe Aufnahmefrequenzen bereit. Die Verstärkungseinrichtung 30 ermöglicht es demgegenüber einerseits die für das Auge 2 zulässigen Strahlungsleistungsgrenzwerte nicht zu überschreiten und andererseits schnellere Sensoren zur Erfassung von Wellenfronten zu verwenden, die eine geringe Lichtempfindlichkeit aufweisen. Insbesondere dient die Verstärkereinrichtung 30 dazu, einem Sensor 32 verstärkte, aus der Messstrahlung 20 resultierende Strahlung bereitzustellen, so dass ein für Signalauswertungen ausreichender Signal-Rausch-Abstand erreicht wird.

Der Sensor 32 ist vorzugsweise ein CMOS-Sensor mit Bildraten von bis zu 500 Bildern pro Sekunde und mehr.

Insbesondere ist vorgesehen, dass der Sensor 32 Messungen mit einer Frequenz von mehr als 100 Hertz ermöglicht. Hierfür sind aufgrund ihrer hohen Bildraten CMOS-Sensoren besonders geeignet.

Der Sensor 32 gibt erfasster Messstrahlung entsprechende Signale aus, die an eine Auswerteeinrichtung 34 weitergeleitet werden. Als Auswerteeinrichtung 34 kann beispielsweise ein digitaler Signalprozessor verwendet werden.

Insbesondere ist es vorgesehen, dass die Linsennordung 28 und der Sensor 32 einen Hartmann-Shack-Sensor darstellen. Dabei wird von der Linsenanordnung 28 ein Punktmuster auf den Sensor 32 abgebildet, das Informationen über Wellenfronten der Messstrahlung enthält.

Zur Steuerung der Vorrichtung von Fig. 1 ist eine Steuereinrichtung 36 vorgesehen. Die Steuereinrichtung 36 kann einen Personal Computer, einen Mikroprozessor und dergleichen umfassen. Die Steuereinrichtung 36 steuert insbesondere den Betrieb der gesamten Vorrichtung von Fig. 1 einschliesslich der Stimuluserzeugungseinrichtung 6 und der Sensoreinrichtung 26.

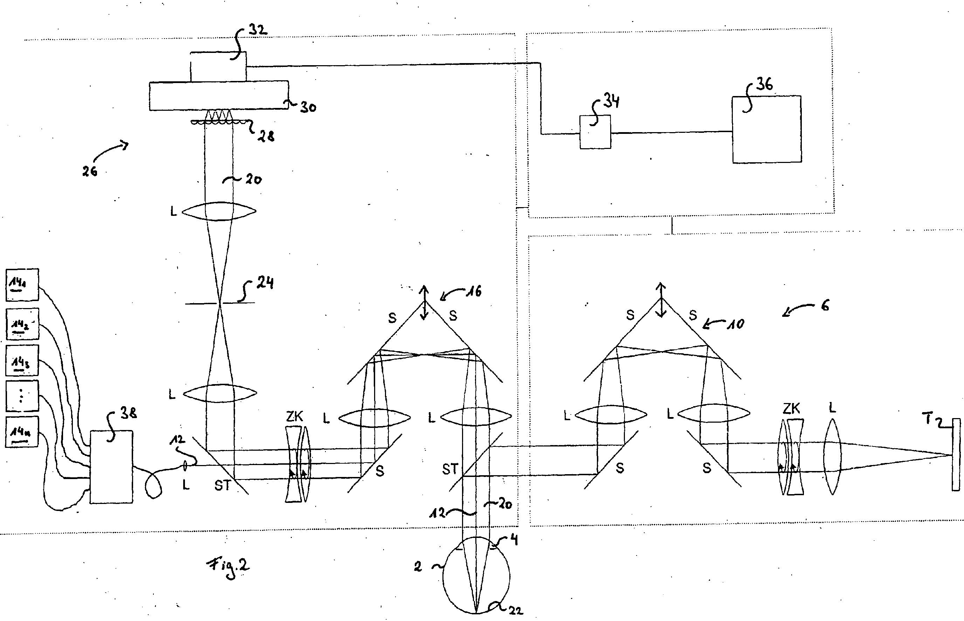

Die in Fig. 2 schematische Darstellung zeigt eine Ausführungsform, die sich von der Ausführungsform gemäss Fig. 1 wie im Folgenden ausgeführt unterscheidet. Bei beiden Ausführungsformen verwendete Komponenten sind mit gleichen Bezugszeichen angegeben.

Die auch bei der Ausführungsform von Fig. 2 vorgesehene Bewegbarkeit der Spiegel- oder Prismenanordnungen 10 und 16 kann bei den im Folgenden beschriebenen Messungen, insbesondere auch bei statischen Messungen, zur Vorkompensation von Fehlsichtigkeit verwendet werden.

Die Ausführungsform von Fig. 2 verwendet zur Erzeugung der Messstrahlung 12 mehrere Strahlungsquellen 141 - 14n unterschiedlicher, diskreter Wellenlängen. In Fig. 2 sind beispielhaft fünf solcher Strahlungsquellen 14 dargestellt. Beispielsweise können die Strahlungsquellen 14 Strahlung in einem Wellenlängenbereich über den gesamten sichtbaren Bereich bis in den nahen Infrarotbereich hinein abgeben. Die Strahlungsquellen 14 können z. B. Laser, Laserdioden und/oder SLDs sein.

Von den Strahlungsquellen 14 abgegebene Strahlung wird zu einer Schalteinrichtung 38 übertragen. Die Übertragung von Strahlung der Strahlungsquellen 14 zu der Schalteinrichtung 38 kann beispielsweise über optische Faserleiter erfolgen.

Die Schalteinrichtung 38, beispielsweise in Form eines sogenannten Faserschalters , wird mit einer Schaltfrequenz betrieben, um Strahlung der Strahlungsquellen 14 zu unterschiedlichen Zeitpunkten und/oder in unterschiedlichen zeitlichen Abständen und/oder für unterschiedliche Zeitdauern als Messstrahlung 12 auszugeben. Die Abfolge, in der Strahlung der Strahlungsquellen 14 als Messstrahlung 12 abgegeben wird, kann beispielsweise bei der kleinsten (grössten) Wellenlänge beginnen und bis zur grössten (kleinsten) Wellenlänge fortschreiten, um dann wieder mit der kleinsten (grössten) Wellenlänge zu beginnen. Es ist auch möglich, dass die Abfolge, in der Strahlung der Strahlungsquellen 14 als Messstrahlung 12 abgegeben wird, in beliebiger, chaotischer Abfolge durchgeführt wird.

Eine mögliche Anwendung der Vorrichtung von Fig. 2 ist die Erfassung der chromatischen Aberration des Auges 2 bzw. der Linse 4. Hierfür kann dem Auge 2 von der Stimuluserzeugungseinrichtung 6 ein als ortsfest wahrnehmbarer Stimulus bereitgestellt werden. Dabei werden dem Auge 2 Strahlungen der Strahlungsquellen 14 über die Schalteinrichtung 38 als Messstrahlung 12 zugeführt. Die Schalteinrichtung 38 wird mit einer Schaltfrequenz betrieben, die hoch genug ist, um von einem statischen Zustand des Auges 2 und insbesondere der Linse 4 ausgehen zu können. Auch wenn die Stimuluserzeugungseinrichtung 6 einen ortsfest erscheinenden Stimulus bereitstellt, ist das Auge bei Fixierung eines solchen Stimulus gewissen in geringem Masse dynamischen Änderungen unterworfen, wie zum Beispiel Mikrosakkaden. Um den Einfluss solcher Änderungen auszuschliessen, wird die Schalteinrichtung 38 mit einer entsprechend hohen Schaltfrequenz betrieben. Beispielsweise kann die Schaltfrequenz 100 Hertz betragen.

Messstrahlung 20 nach Wechselwirkung mit dem Auge 2 bzw. der Linse 4 wird, wie oben unter Bezugnahme auf Fig. 1 beschrieben, der Sensoreinrichtung 26 zugeführt. Hier umfasst die Messstrahlung 20 Strahlungen unterschiedlicher Wellenlängen, nämlich Wellenlängen der Strahlungsquellen 14. Um Wechselwirkungen der Messstrahlung 12 jeweils für die unterschiedlichen Wellenlängen der Strahlungsquellen 14 zu erfassen, wird die Sensoreinrichtung 26 und insbesondere der Sensor 32 bei der Vermessung von Wellenfronten mit einer Abtastfrequenz (entsprechend der Messung einer Wellenfront) betrieben, die wenigstens so gross wie die Schaltfrequenz der Schaltreinrichtung 38 ist.

Aufgrund der Wahl der Schaltfrequenz der Schalteinrichtung 38 geben von der Sensoreinrichtung 26 erfasste Wellenfronten der Messstrahlung 20 für die unterschiedlichen Wellenlängen die chromatische Aberration des Auges 2 bzw. der Linse 4 an.

Wird die Sensoreinrichtung 26 mit einer ausreichend hohen Abtastfrequenz betrieben, ist es möglich, die Vorrichtung von Fig. 2 sowohl zur Erfassung akkommodationsabhäniger Charakteristika des Auges 2 als auch zur Messung von dessen chromatischer Aberration zu verwenden. Für eine solche Anwendung ist vorgesehen, die Stimuluserzeugungseinrichtung 6 so zu betreiben, dass durch in unterschiedlichen Abständen erscheinende Stimuli dynamische Akkommodationsvorgänge bewirkt werden. Um dabei auch von Wellenlängen abhängige Charakteristika des Auges 2 zu ermitteln, wird die Schalteinrichtung 38 mit einer derart hohen Schaltfrequenz betrieben, dass für wenigstens einen Akkommodationszustand, vorteilhafterweise für mehrere oder jeden Akkommodationszustand, hinsichtlich der unterschiedlichen Wellenlängen von einem statischen Zustand des Auges ausgegangen werden kann.

Um die dann in der Messstrahlung 20 vorliegende Information zu detektieren, sind die Sensoreinrichtung 26 und insbesondere der Sensor 32 mit einer Abtastfrequenz zu betreiben, die einem ganzzahligen Vielfachen des Produktes der Frequenzen entspricht, mit denen die Stimuluserzeugungseinrichtung 6 und die Schalteinrichtung 38 betrieben werden.

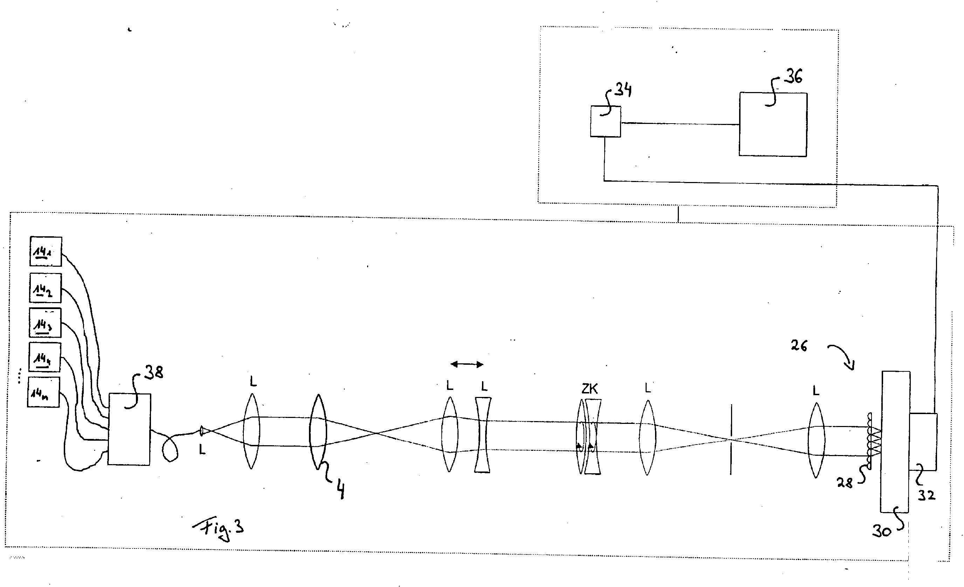

Die in Fig. 3 schematisch dargestellte Ausführungsform wird zur Messung der Dispersion einer Linse für ein Auge verwendet. Die Linse 4 kann beispielsweise eine Kontaktlinse oder intraokulare Linse sein. Vergleichbar zu der Ausführungsform von Fig. 2 umfasst die Ausführungsform von Fig. 3 Strahlungsquellen 141 - 14n, die Strahlung unterschiedlicher Wellenlängen abgeben. Die in diesem Zusammenhang unter Bezugnahme auf Fig. 2 gemachten Ausführungen gelten hier entsprechend. Dies gilt auch für die Strahlung von den Strahlungsquellen 14 erhaltende Schalteinrichtung 38. Von der Schalteinrichtung 38 abgegebene Messstrahlung 12 wird direkt oder über zwischen der Schalteinrichtung 38 und der Linse 4 angeordnete Optiken, die in Fig. 3 lediglich zur Veranschaulichung eine Austrittslinse L und eine Linse Ls zur Strahlaufweitung umfassen, der Linse 4 zugeführt. Messstrahlung 20, die sich aus Wechselwirkung der Messstrahlung 12 mit der Linse 4 ergibt, wird über beispielhaft Linsen L, und einem Zylinderkompensator ZK (insbesondere zur Vorkomensation) sowie eine Blende Bl mit nachgeschalteter Linse L umfassende Optiken einer Sensoreinrichtung 26 zugeführt.

Die Sensoreinrichtung 26 ist mit den Sensoreinrichtungen 26 von Fig. 1 und 2 im Wesentlichen vergleichbar. Insbesondere gilt dies für die Abtastraten, mit denen die Sensoreinrichtung 26 betrieben wird.

Mit der Ausführungsform von Fig. 3 ist es möglich, die Dispersion, d.h. die wellenlängenabhängige Brechung von Licht durch die Linse 4 zu ermitteln. Auch im Fall von Kontaktlinsen und intraokularen Linsen wird die Sensoreinrichtung 26 mit einer wie oben ausgeführt hohen Abtastfrequenz zu betreiben, was den Vorteil hat, dass die Überprüfung wellenlängenabhängiger Eigenschaften von Linsen für Augen, beispielsweise im Rahmen der industriellen Fertigung von Kontaktlinsen, besonders schnell durchgeführt werden kann. Mit der Vorrichtung von Fig. 3 ist es ferner möglich, die Qualitätskontrolle zu verbessern.

Bei der obigen Beschreibung der Ausführungsformen von Fig. 1 und 2 wird davon ausgegangen, dass es sich bei der Linse 4 des Auges 2 um dessen eigene Linse handelt. Es ist allerdings auch möglich, die Ausführungsformen von Fig. 1 und 2 zur Messung an einem Auge zu verwenden, das zusätzlich eine Kontaktlinse aufweist oder bei dem eine intraokulare Linse eingesetzt ist. Auch ist es möglich, die Vorrichtung von Fig. 1 und 2 zur Messung an einem Auge zu verwenden, das einer refraktiven chirurgischen Behandlung unterzogen wird.

## Patentansprüche

1. Vorrichtung zur Messung von Wellenfronten, die von einer Linse für ein Auge erzeugt werden, mit:
- mehrere Strahlungsquellen (14) zur Abgabe von auf die Linse (4) zu richtender Messstrahlung (12) von unterschiedlichen, diskreten Wellenlängen¹ und
- einer Sensoreinrichtung (26) zur Erfassung von Wellenfronten von einfallender Messstrahlung (20) nach Wechselwirkung mit der Linse (4) mit einem Sensor (32), wobei
¹ Vgl. Seite 8, Absatz [0041]
- die Sensoreinrichtung (26) die einfallende Messstrahlung (20) mit einer Abtastfrequenz erfasst, die wenigstens so groß wie die Frequenz ist, mit der Wellenfrontänderungen in der einfallenden Messstrahlung (20) auftreten,
- einer Verstärkungseinrichtung (30), zur Verstärkung der auf den Sensor (32) gelangenden Messstrahlung, bevor die Messstrahlung von dem Sensor (32) hochfrequent erfasst wird, wobei die Verstärkungseinrichtung ein Bildverstärker ist, - wobei die Sensoreinrichtung (26) eine Abtastfrequenz von wenigstens 70 Hertz aufweist,
- einer Stimuluserzeugungseinrichtung (6) zur Erzeugung eines dynamische Änderungen der Linse bewirkenden Stimulus, wobei die Abtastfrequenz wenigstens so groß wie die Frequenz der zu bewirkenden Änderungen ist²,
² Vgl. Anspruch 3
- wobei die mehreren Strahlungsquellen (14) ausgangsseitig mit einer Schalteinrichtung (38) verbunden sind, die eine Schaltfrequenz aufweist, wobei die Abtastfrequenz wenigstens so groß wie die Schaltfrequenz ist, und³
³ vgl. Anspruch 5
- wobei die Abtastfrequenz einem ganzzahligen Vielfachen des Produkts der Frequenz der zu bewirkenden Änderungen und der Schaltfrequenz entspricht⁴.
⁴ Vgl. Seite 9, Absatz [0048]

2. Vorrichtung nach Anspruch 1, wobei die Strahlungsquelle (14) Messstrahlung (12) mit einer Strahlungsabgabefrequenz und sich ändernder Wellenlänge abgibt, derart, dass die Abtastfrequenz wenigstens so groß wie die Strahlungsabgabefrequenz ist.

3. Vorrichtung nach Anspruch 1 oder 2, mit
- einem Badaloptometer als die Stimuluserzeugungseinrichtung (6).

4. Vorrichtung nach einem der vorherigen Ansprüche, bei der
- die Sensoreinrichtung (26) einen optischen Sensor (32) umfasst und/oder
- die Sensoreinrichtung (26) einen CMOS-Sensor als optischen Sensor (32) umfasst und/oder
- die Sensoreinrichtung (26) eine Verstärkungseinrichtung (30) zur Verstärkung einfallender Messstrahlung (20) umfasst und/oder
- die Sensoreinrichtung (26) einen Bildverstärker als Verstärkungseinrichtung (30) zur Verstärkung einfallender Messstrahlung (20) umfasst und/oder
- die Sensoreinrichtung (26) eine Verstärkungseinrichtung (30) zur Verstärkung einfallender Messstrahlung (20) umfasst, wobei die Verstärkungseinrichtung (30) zur Verstärkung einfallender Messstrahlung (20) vor deren Erfassung mit der Abtastrate angeordnet ist, und/oder
- die Sensoreinrichtung (26) eine Linsenanordnung (28) umfasst und/oder
- die Linsenanordnung (28) in Abhängigkeit einer gewünschten Auflösung und/oder einer gewünschten Dynamik der Sensoreinrichtung (26) ausgelegt ist und/oder
- die Strahlungsquelle (14) zur Abgabe von Messstrahlung (12) mit einer für die Linse (4) und/oder das Auge (2) vorgegebenen maximalen Strahlungsleistung ausgelegt ist und/oder
- die Strahlungsquelle (14) wenigstens eine Laserstrahlenquelle, z.B. ein Laser, eine Laserdiode und/oder eine Superluminiszenzdiode ist, und/oder
- die Strahlungsquelle (14) zur Abgabe von Messstrahlung mit wenigstens einer Wellenlänge im Bereich zwischen 400 nm und 1000 nm ausgelegt ist.

5. Vorrichtung nach einem der vorherigen Ansprüche, bei der
- die Strahlungsquelle (14) ausgangsseitig mit einem Faserschalter und/oder einem Faserkoppier als Schalteinrichtung (38) verbunden ist, die eine Schaltfrequenz aufweist, wobei die Abtastfrequenz wenigstens so groß wie die Schaltfrequenz ist.

6. Verfahren zur Messung von Wellenfronten, die von einer Linse für ein Auge erzeugt werden, mit folgenden Schritten:
- Aussenden von Messstrahlung von mehreren Strahlungsquellen (14) von unterschiedlichen, diskreten Wellenlängen auf die Linse und
- Erfassen von Wellenfronten von Messstrahlung nach Wechselwirkung mit der Linse, wobei
- die Erfassung von Messstrahlung nach Wechselwirkungen mit der Linse mit einem Sensor (32) und mit einer Abtastfrequenz erfolgt, die wenigstens so groß wie die Frequenz ist, mit der Wellenfrontänderungen in der Messstrahlung nach Wechselwirkung mit der Linse auftreten,
wobei mittels einer Verstärkungseinrichtung (30), die auf den Sensor (32) gerichtete Messstrahlung verstärkt wird, bevor die Messstrahlung von dem Sensor hochfrequent erfasst wird, wobei die Verstärkung mit einem Bildverstärker erfolgt, und
die Sensoreinrichtung (26) eine Abtastfrequenz von wenigstens 70 Hertz aufweist,
- Erzeugen eines dynamische Änderungen der Linse bewirkenden Stimulus, wobei die Abtastfrequenz wenigstens so groß wie die Frequenz der zu bewirkenden Änderungen ist,
- wobei die mehreren Strahlungsquellen (14) ausgangsseitig mit einer Schalteinrichtung (38) verbunden sind, die eine Schaltfrequenz aufweist, wobei die Abtastfrequenz wenigstens so groß wie die Schaltfrequenz ist, und
- wobei die Abtastfrequenz einem ganzzahligen Vielfachen des Produkts der Frequenz der zu bewirkenden Änderungen und der Schaltfrequenz entspricht.

7. Verfahren nach Anspruch 6 zur Messung dynamischer Akkommodationsänderungen der Linse eines Auges.

8. Verfahren nach Anspruch 6 oder 7 zur Messung chromatischer Aberrationen eines. Auges.

9. Verfahren nach einem der Ansprüche 6 bis 8 zur Messung der Dispersion einer Kontaktlinse oder einer intraokularen Linse für ein Auge.

10. Verfahren nach einem der Ansprüche 6 bis 9 zur Messung optischer Einflüsse bei Bewegungen einer Kontaktlinse oder intraokularen Linse relativ zum Übrigen eines damit versehenen Auges.

11. Verfahren nach einem der Ansprüche 6 bis 10 zur Messung optischer Einflüsse von Tränenfilmänderungen.

12. Verfahren nach einem der Ansprüche 6 bis 11 zur Erfassung und/oder Korrektur von Abberationen einer intraokularen Linse eines Auges.

## Claims

1. An apparatus for measuring wavefronts, which are generated by a lens for an eye, comprising:
- plural radiation sources (14) for emitting a measurement radiation (12) to be directed onto the lens (4) having different, discrete wavelengths; and
- sensor means (26) for detecting wavefronts of incident measurement radiation (20) after interaction with the lens (4) with a sensor (32), wherein
- the sensor means (26) detects the incident measurement radiation (20) with a sampling frequency, which is equal or greater than the frequency, with which wavefront changes occur in the incident measurement radiation (20),
- amplification means (30) for amplification of the measurement radiation incident on the sensor (32), before the measurement radiation is high frequency-detected by the sensor (32), wherein the amplification means is an image amplifier,
- wherein the sensor means (26) has a sampling frequency of at least 70 Hertz,
- stimulus generating means (6) for generating a stimulus causing a dynamic change of the lens, wherein the sampling frequency is equal to or greater than the frequency of the changes to be caused,
- wherein the plural radiation sources (14) are connected to switching means (38) on their output side, which switching means have a switching frequency, wherein the sampling frequency is equal to or greater than the switching frequency, and
- wherein the sampling frequency corresponds to an integer multiple of the product of the frequency of the changes to be caused by the switching frequency.

2. The apparatus according to claim 1, wherein the radiation source (14) emits measurement radiation (12) having a radiation emission frequency and a changing wavelength such that the sampling frequency is equal to or greater than the radiation emission frequency.

3. The apparatus according to claim 1 or 2, comprising
- a badaloptometer as the stimulus generating means (6).

4. The apparatus according to any one of the preceding claims, wherein
- the sensor means (26) comprises an optical sensor (32), and/or
- the sensor means (26) comprises a CMOS sensor as optical sensor (32), and/or
- the sensor means (26) comprises an amplification means (30) for amplifying incident measurement radiation (20), and/or
- the sensor means (26) comprises an image amplifier as amplifying means (30) for amplifying incident measurement radiation (20), and/or
- the sensor means (26) comprises amplification means (30) for amplifying incident measurement radiation (20), wherein the amplification means (30) is arranged for amplifying incident measurement radiation (20) prior to detection with the sampling rate, and/or
- the sensor means (26) comprises a lens arrangement (28), and/or
- the lens arrangement (28) is arranged depending on a desired resolution and/or a desired dynamic of the sensor means (26), and/or
- the radiation source (14) for emission of measurement radiation (12) is arranged with a maximum radiation power prescribed for the lens (4) and/or the eye (2), and/or
- the radiation source (14) is at least one laser beam source, e.g. a laser, a laser diode and/or a superluminescence diode, and/or
- the radiation source (14) for emission of measurement radiation is arranged with at least a wavelength in a range between 400 nm and 1000 nm.

5. The apparatus according to any one of the preceding claims, wherein
- the radiation source (14) is connected to a faser switch and/or a faser coupler as switching means (38) at its output side, which switching means has a switching frequency, wherein the sampling frequency is equal to or greater than the switching frequency.

6. A method for measuring of wavefronts, which are generated by a lens for an eye, comprising the steps of:
- emitting measurement radiation from plural radiation sources (14) having different discrete wavelengths onto the lens, and
- detecting wavefronts of measurement radiation after interaction with the lens, wherein
- the detection of measurement radiation is effected after interactions with the lens with a sensor (32) and with a sampling frequency, which is equal to or greater than the frequency, with which wavefront changes in the measurement radiation occur after interaction with the lens,
wherein, by means of amplification means (30), the measurement radiation directed onto the sensor (32) is amplified before the measurement radiation is high frequency-detected by the sensor, wherein the amplification is effected by an image amplifier, and the sensor means (26) has a sampling frequency of at least 70 Hertz,
- generating a stimulus causing dynamic changes of the lens, wherein the sampling frequency is equal to or greater than the frequency of the changes to be caused,
- wherein the plural radiation sources (14) are connected to a switching means (38) on their output sides, wherein the switching means has a switching frequency, wherein the sampling frequency is equal to or greater than the switching frequency, and
- wherein the sampling frequency corresponds to an integer multiple of the product of the frequency of the changes to be caused by the switching frequency.

7. The method according to claim 6 for measuring dynamic accommodation changes of the lens of an eye.

8. The method according to claim 6 or 7 for measuring chromatic aberrations of an eye.

9. The method according to any one of claims 6 to 8 for measuring the dispersion of a contact lens or of an intraocular lens for an eye.

10. The method according to any one of claims 6 to 9 for measuring optical influences when a contact lens or an intraocular lens is moved relative to the remainder of an eye equipped with the contact lens or the intraocular lens.

11. The method according to any one of claims 6 to 10 for measuring optical influences by tear film changes.

12. The method according to any one of claims 6 to 11 for detecting and/or correction of aberrations of an intraocular lens of an eye.

## Revendications

1. Dispositif pour la mesure de fronts d'onde produits par une lentille pour un oeil, comprenant
- plusieurs sources de rayonnement (14) destinées à émettre un rayonnement de mesure (12) de différentes longueurs d'ondes discrètes à diriger sur la lentille (4) et
- un dispositif de détection (26) pourvu d'un capteur (32) pour détecter des fronts d'onde du rayonnement de mesure incident (20) après interaction avec la lentille (4),
- ledit dispositif de détection (26) détectant le rayonnement de mesure incident (20) à l'aide d'une fréquence de balayage qui est pour le moins égale à la fréquence à laquelle se produisent des modifications de fronts d'onde dans le rayonnement de mesure incident (20),
- un dispositif d'amplification (30) servant à amplifier le rayonnement de mesure atterrissant sur ledit capteur (32) avant que le rayonnement de mesure soit détecté à haute fréquence, ledit dispositif d'amplification (30) consistant en un amplificateur d'images, - le dispositif de détection (26) présentant une fréquence de balayage d'au moins 70 Hertz,
- un dispositif générateur de stimulus (6) pour générer un stimulus provoquant des modifications dynamiques de la lentille, la fréquence de balayage étant pour le moins égale à la fréquence des modifications à provoquer,
- les multiples sources de rayonnement (14) étant reliées, côté sortie, à un dispositif de commutation (38) présentant une fréquence de commutation, la fréquence de balayage étant pour le moins égale à ladite fréquence de commutation, et
- la fréquence de balayage correspondant à un multiple entier du produit de la fréquence des modifications à provoquer et de la fréquence de commutation.

2. Dispositif selon la revendication 1, la source de rayonnement (14) émettant un rayonnement de mesure (12) à une fréquence de balayage et à une longueur d'onde variable de sorte telle que la fréquence de balayage est pour le moins égale à celle de la fréquence d'émission de rayon.

3. Dispositif selon la revendication 1 ou la revendication 2, comprenant
- un optomètre de Badal en tant que dispositif générateur de stimulus (6).

4. Dispositif selon l'une des revendications précédentes, dans le cadre duquel
- le dispositif de détection (26) comporte un capteur optique (32), et/ou
- le dispositif de détection (26) comporte un capteur CMOS en tant que capteur optique (32), et/ou
- le dispositif de détection (26) comporte un dispositif d'amplification (30) servant à amplifier le rayonnement de mesure incident (20), et/ou
- le dispositif de détection (26) comporte un amplificateur d'images en tant que dispositif d'amplification (30) servant à amplifier le rayonnement de mesure incident (20), et/ou
- le dispositif de détection (26) comporte un dispositif d'amplification (30) servant à amplifier le rayonnement de mesure incident (20), le dispositif d'amplification (30) servant à amplifier le rayonnement de mesure incident (20) étant disposé en amont de la détection de ce dernier à l'aide de la fréquence de balayage, et/ou
- le dispositif de détection (26) comporte un agencement de lentilles (28), et/ou
- ledit agencement de lentilles (28) est conçu en fonction d'une résolution souhaitée et/ou d'une dynamique souhaitée du dispositif de détection (26), et/ou
- la source de rayonnement (14) destinée à émettre un rayonnement de mesure (12) est conçue pour une puissance de rayonnement maximale prédéfinie pour la lentille (4) ou pour l'oeil (2), et/ou
- la source de rayonnement (14) consiste pour le moins en une source de rayon laser, telle que par exemple un laser, une diode laser et/ou une diode superlumines-cente, et/ou
- la source de rayonnement (14) destinée à émettre un rayonnement de mesure ayant au moins une longueur d'onde comprise entre 400 nm et 1000 nm.

5. Dispositif selon l'une des revendications précédentes, dans le cadre duquel
- la source de rayonnement (14) est reliée, côté sortie, à un commutateur à fibre et/ou à un coupleur à fibre en tant que dispositif de commutation (38) présentant une fréquence de commutation, la fréquence de balayage étant pour le moins égale à ladite fréquence de commutation.

6. Procédé permettant la mesure de fronts d'ondes produits par une lentille pour un oeil, comportant les étapes suivantes :
- l'émission par plusieurs sources de rayonnement (14) d'un rayonnement de mesure de différentes longueurs d'ondes discrètes sur la lentille (4) et
- la détection de fronts d'onde du rayonnement de mesure après interaction avec la lentille,
- la détection d'un rayonnement de mesure après interaction avec la lentille étant effectuée à l'aide d'un capteur (32) et d'une fréquence de balayage qui est pour le moins égale à la fréquence à laquelle se produisent des modifications de fronts d'onde dans le rayonnement de mesure après interaction avec la lentille,
le rayonnement de mesure dirigé sur le capteur (32) étant amplifié au moyen d'un dispositif d'amplification (30) avant détection à haute fréquence du rayonnement de mesure par le capteur, l'amplification étant effectuée à l'aide d'un amplificateur d'images, et le dispositif de détection (26) présentant une fréquence de balayage d'au moins 70 Hertz,
- la production d'un stimulus provoquant des modifications dynamiques de la lentille, la fréquence de balayage étant pour le moins égale à la fréquence des modifications à provoquer,
- les multiples sources de rayonnement (14) étant reliées, côté sortie, à un dispositif de commutation (38) présentant une fréquence de commutation, la fréquence de balayage étant pour le moins égale à la fréquence de commutation, et
- la fréquence de balayage correspondant à un multiple entier du produit de la fréquence des modifications à provoquer et de la fréquence de commutation.

7. Procédé selon la revendication 6 permettant de mesurer les modifications d'accommodation de la lentille d'un oeil.

8. Procédé selon la revendication 6 ou la revendication 7 permettant de mesurer les aberrations chromatiques d'un oeil.

9. Procédé selon l'une des revendications 6 à 8 permettant de mesurer la dispersion d'un verre de contact ou d'une lentille intraoculaire pour un oeil.

10. Procédé selon l'une des revendications 6 à 9 permettant de mesurer les influences optiques lors du mouvement d'un verre de contact ou d'une lentille intraoculaire par rapport au reste de l'oeil qui en est pourvu.

11. Procédé selon l'une des revendications 6 à 10 permettant de mesurer les influences optiques des modifications du film lacrymal.

12. Procédé selon l'une des revendications 6 à 11 permettant de détecter et/ou de corriger des aberrations d'une lentille intraoculaire d'un oeil.
